# EUROPEAN PATENT SPECIFICATION

(11) **EP 1 292 228 B1**
(45) Date of publication and mention of the grant of the patent: **24.08.2005**
(21) Application number: 01915938.3
(22) Date of filing: 21.02.2001
(51) Int. Cl.: A61B 7/04, H04R 1/46, G01H 11/08

(54) **TWO-WAY MECHANO-ELECTRICAL TRANSDUCER**
MECHANO-ELEKTRISCHER ZWEIWEGWANDLER
TRANSDUCTEUR MECANO-ELECTRIQUE BIDIRECTIONNEL

(30) Priority: 23.06.2000 US 213477 P
(43) Date of publication of application: 19.03.2003
(73) Proprietor: Vibrotron AS, 1392 Vettre (NO)
(72) Inventor: ORTEN, Birger, N-6012 Alesund (NO)
(74) Representative: Brunner, Michael John
(86) International application number: PCT/NO2001/000064
(87) International publication number: WO 2002/000117

(56) References cited:
- EP-A2- 0 118 329
- GB-A- 2 166 022
- JP-A- 7 143 765
- US-A- 4 727 279

## Description

The present invention relates to applying and sensing acoustical or mechanical vibration, together with supply and delivery of electrical signals corresponding to a parameter of a vibrational state. More in particular, the invention relates to a mechano-electrical transducer for applying and sensing vibration together with supply and delivery of at least one electrical signal that corresponds to the applied, respectively sensed vibration.

Transducers between mechanical and electrical energy (vibrations, force, acceleration) have many uses, and exist in many embodiments. Usually, two or three separate transducers are employed for instance to sense acceleration in three orthogonal directions, by letting massive bodies, suspended in spring systems, move relative to respective reference frameworks.

From GB 2.055.018 and EP 118.329 are previously known transducers with a massive body suspended centrally in piezoelectric, flexible "lamellas" or in piezoelectric filaments, for application in seismic and acoustical detection.

Related art can also be found in GB 2.166.022, which publication shows a transducer in the form of a loudspeaker, i.e. with conversion from electrical to acoustical signals, and using a massive body that is suspended centrally in a piezoelectric thin loudspeaker diaphragm that may be divided in several lamella-like areas by means of radial cuts. Since it is ordinarily known that a loudspeaker can also be used as a microphone, the last mentioned publication must be regarded as stating a two-way transducer. However, the point of the central body in the publication, is that the useful frequency range of the loudspeaker can be lowered by adding the center mass.

The present invention aims at providing a two-way transducer which, better than previously known solutions, is able to operate with a directional effect and provide improved emission and detection, particularly in connection with echo measurements in biological tissue.

Hence, in accordance with the invention there is provided a two-way mechano-electrical transducer such as defined precisely in the appended claim 1. Preferable embodiments of the invention appear from the attached dependent claims.

In the following, the invention shall be illuminated further by going through exemplary embodiments, and in this connection it is also referred to the appended drawings, in which
fig. 1 shows a first embodiment of the transducer in accordance with the invention, with transmission and reception activity in suspension sectors,
fig. 2 shows an embodiment with an active center body,
figs. 3a and 3b show a first embodiment with a center body tautening structure,
figs. 4a and 4b show a second such embodiment, and
fig. 5 shows schematically an embodiment with signal phasing.

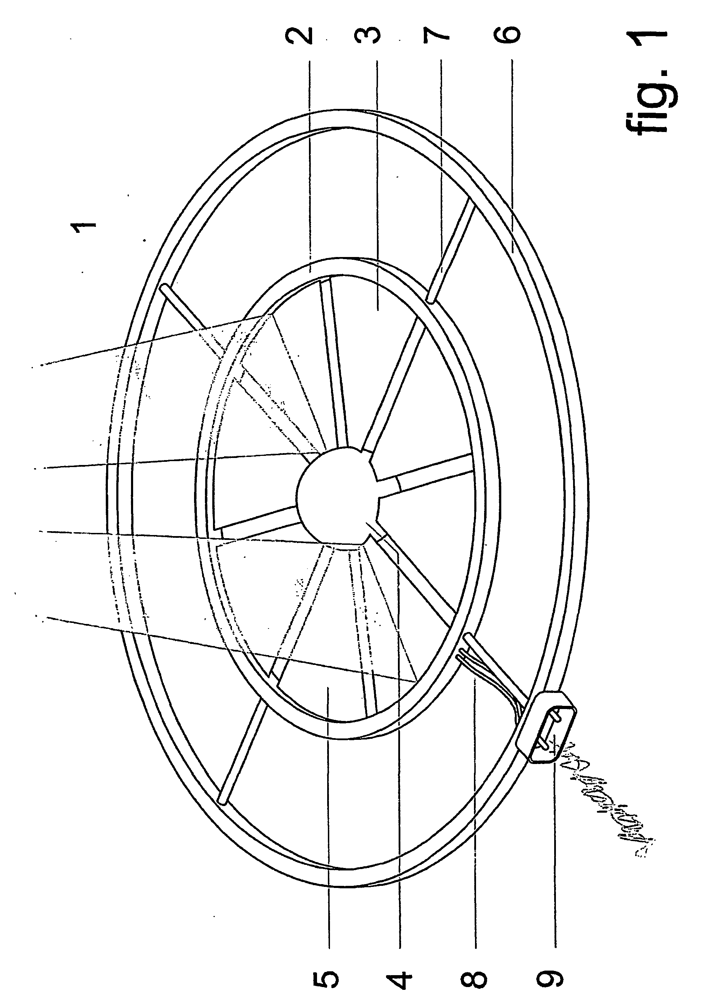

In fig. 1 appears a first embodiment of the transducer 1 in accordance with the invention. In an annular framework 2, a center body 4 is suspended in a flat suspension structure 3 having the form of a number of elastic sheets 5 in sector form, in this case eight such sectors 5.

In the embodiment appearing in fig. 1, the framework 2 is also suspended in an outer frame 6 by means of an outer elastic suspension structure in the form of elastic strings 7, but such an outer frame 6 with an outer suspension structure is not obligatory for the invention.

Signal conductors 8 lead to and from sectors 5, and possibly to and from the center body 4, and a connector 9 is arranged on the outer frame 6. The conductors 8 are not shown in detail further inside the framework 2, but lead to and from each respective sector 5, and possibly to and from the center body 4.

Each sector sheet 5 can be attached at the outer edge between two parts of ring 2, and in the inner "point" attached between two semi-spheres constituting the center body 4. The sector sheets 5 are made for instance of PVDF (polyvinylidene fluoride), which is a material having piezoelectric characteristics, and which is able to convert an electrical input signal to a vibration that may propagate to a medium in front of (above) the transducer, for example body tissue, and impinging vibration waves hitting a sector (or more sectors), to electrical output signals. Each sector 5 is separately addressable with separate conductors 8.

In this first embodiment, the center body 4 is simply a massive body which, when the transducer is mounted as a front part of for instance a handheld examining unit, will engage e.g. the skin surface of a patient, in such a manner that the central part of the transducer will be pushed rearwardly. This causes the sectors 5 to be somewhat angled relative to the unloaded position, and in this manner, a focusing effect is obtained, for example as indicated in fig. 1. Further, it is to be noted that with an outer suspension structure 7 of an elastic type, such as shown in fig. 1, the downward flexing will occur also there. In an embodiment without such an outer structure, the angling of the sectors will be more slanted.

With a transducer of the type indicated, it will be possible to transmit vibrations with one sector or some sectors, and at the same time, another sector, or some other sectors, will be able to receive reflected vibrations, that is an echo.

Another advantage is the possibility for removing undesired signals, i.e. noise, by using the receive signals from different sectors in post-processing. It is also possible to couple a noise signal in opposite phase into the signal conductors of for example one particular sector, in order to cancel the noise part of the receive signal of that sector. The noise signal to be coupled in, may come from for example a piezo-element on the outer suspended structure 7, which element will also receive the incoming noise signal, or from a separately arranged sensor.

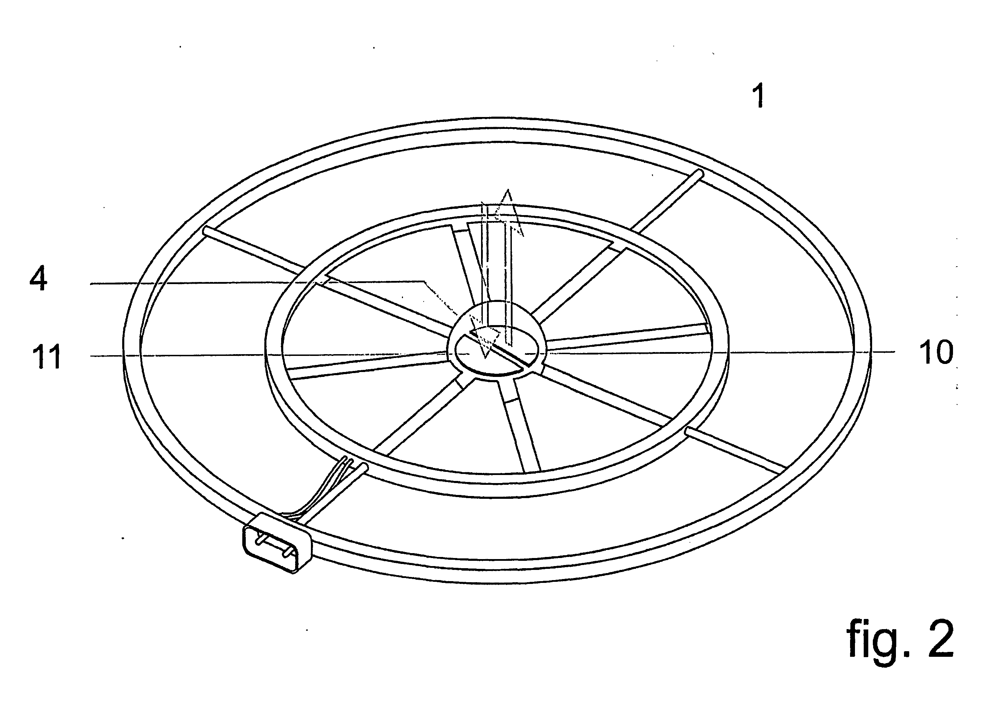

In the embodiment appearing in fig. 2, the center body 4 is "active" also, i.e. the center body itself contains piezo-elements 10 and 11, in this case a piezo-element 10 for transmitting vibrations, and a piezo-element 11 for receiving reflected vibrations. For the rest, the transducer is constructed such as stated in connection with fig.1. Signal conductors to the center body piezo-elements are not shown in fig. 2, however they follow paths on sectors 5 up to the center body 4.

In the shown embodiment, piezo-elements 10 and 11 are cast-in "half moons" of a piezo material. The way of using such an embodiment as discussed here, will be for instance that the center body piezo-element 10 transmits high frequency vibrations, preferably in the range 5-10 MHz (however not limited to this range), in order to make an echo Doppler investigation. Reflected vibrations are picked up by means of element 11.

At the same time as the echo Doppler investigation by means of center body 4, the sectors 5, or some of them, can be used for ordinary auscultation, that is pure listening to acoustical vibrations from a body.

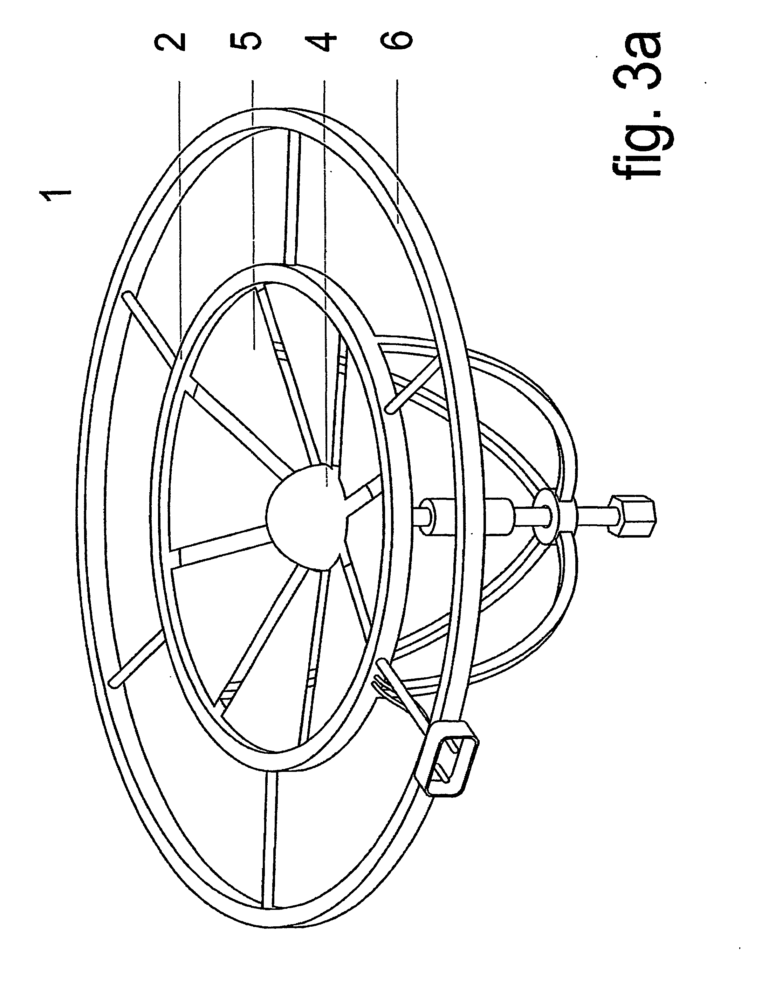

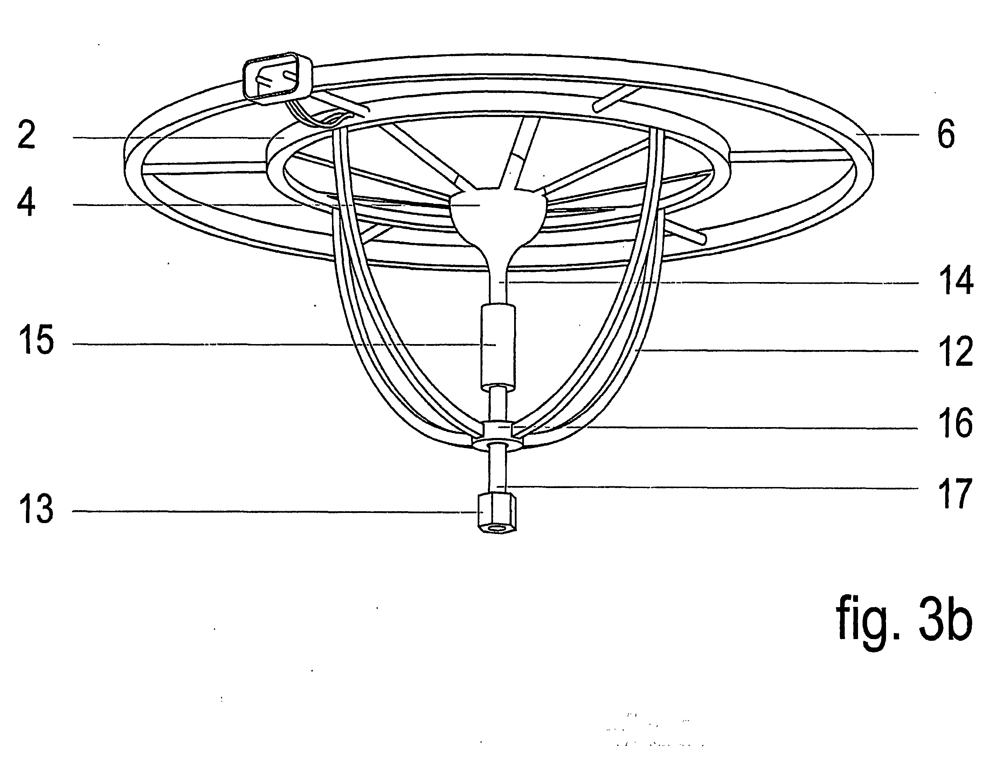

In figs. 3a and 3b appears an embodiment of the transducer in accordance with the invention, in which embodiment it is possible to change and to control the slanted attitude of the sectors (compare the slanted attitude mentioned above, obtained by pressing the center body 4 against a surface). This is achieved by designing the center body 4 with an extended rear part 14 that is gripped/held by a sleeve 15, in such a manner that the sleeve can rotate about a lower (not shown) ball or enlargement on the rear part 14, so that the rear part (and consequently the center body) can be pulled down when the sleeve 15 travels downwards while rotating. Such a rotating downward movement is caused by turning head 13, so that a threaded section inside part 16, held by a number of stays 12, cooperates with threads on bolt 17, so that a vertical motion results. Hence, in such a manner angling and tautening of the sectors 5 can be regulated.

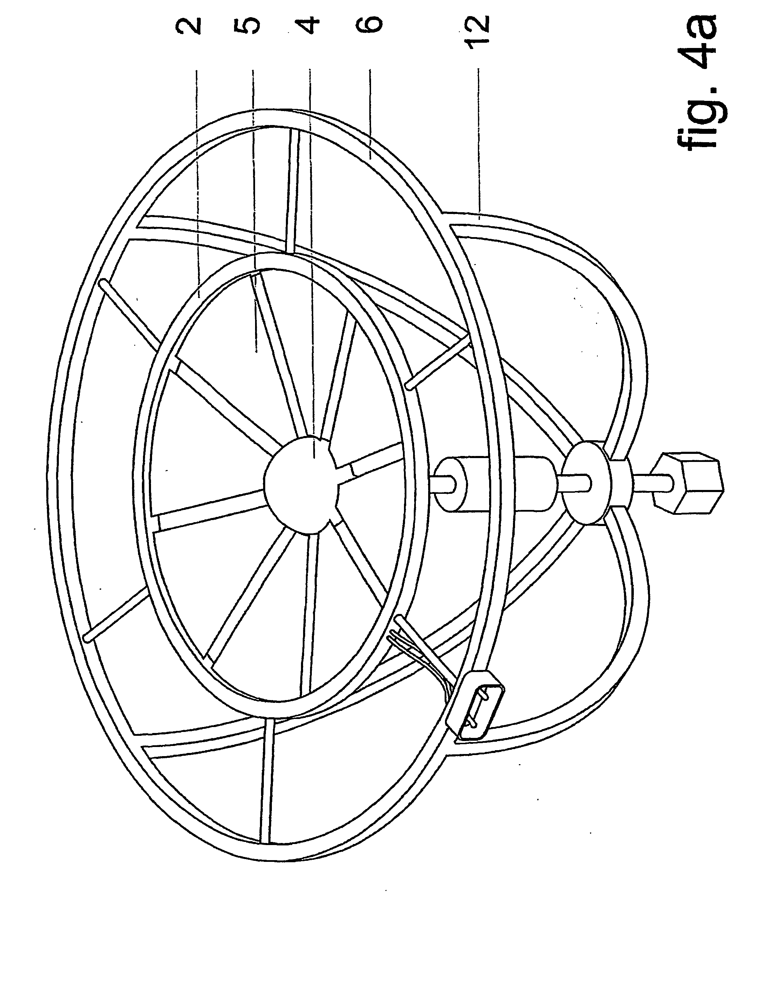

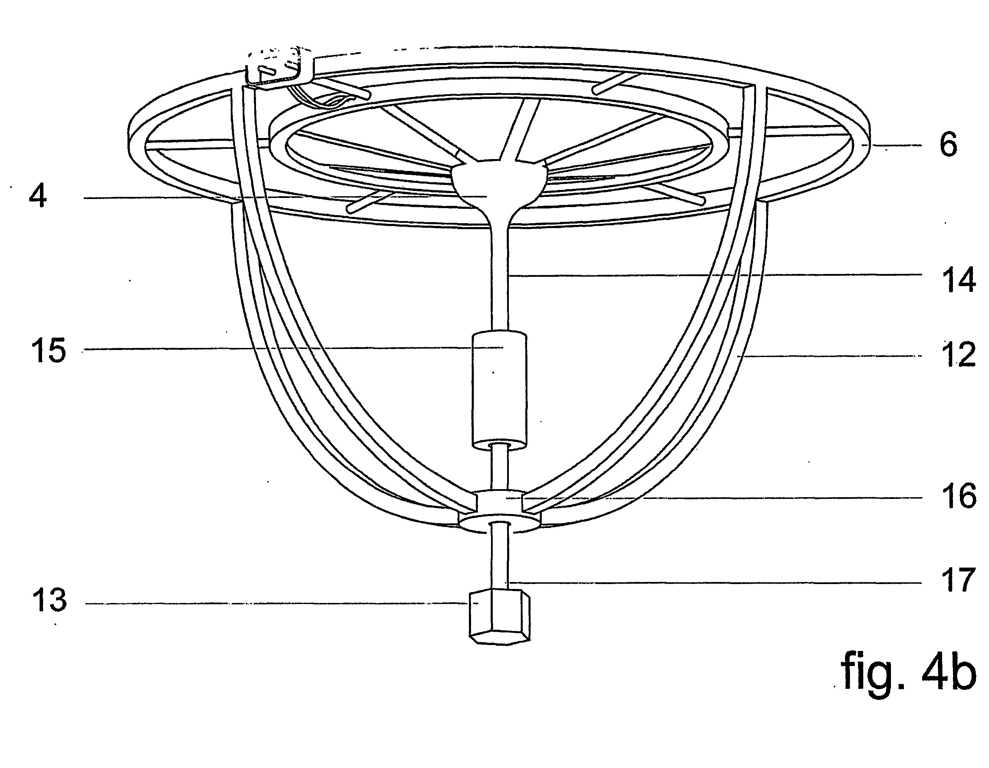

Such as shown in fig. 3a, b, stays 12 are attached to ring 2, so that the tautening will only affect the main part of the transducer with the sectors 5. However, in figs. 4a and 4b appears a variant in which the stays 12 are attached to the outer ring 6. This means that the outer suspension structure also will be tautened and slanted by means of the regulating system 12-17.

The curved shape of the stays 12 is appropriate, but not obligatory.

Moreover, the transducer variants shown in figs. 3 and 4 are intended to operate along the same principles as discussed regarding the embodiments in figs. 1 and 2.

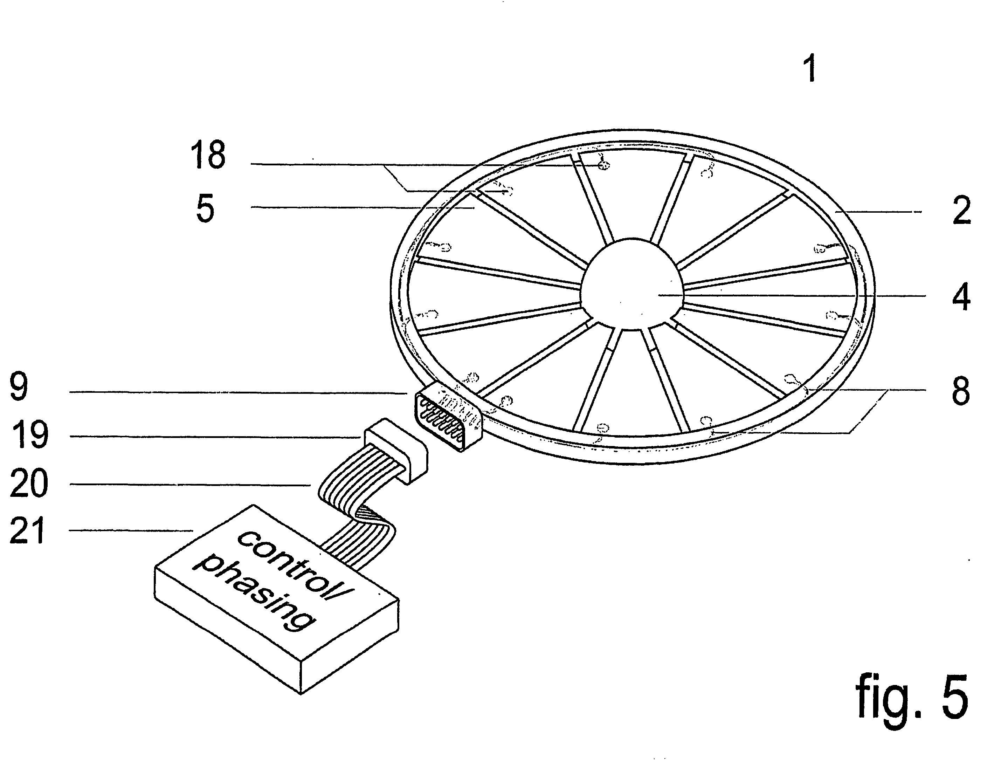

In fig. 5 appears schematically an embodiment in which it is possible to provide, by detailed control of signals applied to the separate piezoelectric sectors 5, further directional control of the mechanical oscillation wave to be transmitted. One utilizes then a principle that is well known from antenna technology, for instance within the field of mobile telephony, in which antennas consisting of several antenna elements, are "fired" with small delays or phase displacements relative to each other, so that constructive interference is obtained in a desired direction out from the total antenna. (This principle is utilized also in receiving/listening, i.e. "listening windows" are opened in the attached receiver electronics in "phased succession", and part signals are added in such a manner that listening is executed effectively in special directions.)

Thus, directional and phased transmission of mechanical vibration waves is performed by making the control unit 21 deliver phase-displaced (and possibly intensity regulated) signals to the sectors 5 via multiwire cable 20 and multi-connectors 19 and 9 and conductors 8, to the contact points 18 on each respective sector, the respective sectors thereby receiving phase controlled signals. (Further detailed control can possibly be obtained by additional radial division of the active piezoelectric sector areas, and with separate signal supply thereto.) In other respects, the signal conductors 8 and contact points 18 are only shown schematically, for instance not all conductors 8 are shown to reach the contact 9, but of course this is the intention. In fig. 5, the transducer has, for simplicity, been shown in an embodiment without an outer frame, but such an outer frame 6 as in the remaining figures, can of course be used also in the "phased" embodiment.

Receiving/listening can of course be performed in accordance with the same phasing principle. A computer/calculating unit in the control unit 21 handles signal phasing both in transmission and reception, in accordance with programmed algorithms.

## Claims

1. Two-way mechano-electrical transducer for emission and pick-up of mechanical vibrations, said transducer comprising a center body (4) suspended in at least one piezoelectric elastic suspension structure (3) which in its turn is mounted in a surrounding framework (2), and signal conductors (8) connected to the suspension structure,
**characterized in that** said suspension structure (3) and said center body (4) are adapted for substantially simultaneous transmission of mechanical vibrations and reception of such vibrations, by sector division of said suspension structure (3), said suspension structure (3) having separate signal conductors for each sector (5).

2. The transducer of claim 1,
**characterized in that** at least one sector (5) of the suspension structure (3) is operative for transmission at the same time as at least one other sector (5) receives an echo signal based on that transmission.

3. The transducer of claim 2,
**characterized in that** it is adapted for use in such a manner that said center body (4) can be forced against a tissue surface during an examination, whereby tension is imparted on said sectors (5) while at the same time the sectors become aslant, in such a manner that signal transmission and signal reception are more directional.

4. The transducer of claim 1,
**characterized in that** the center body (4) is equipped with at least one piezo-element (10, 11) for transmission and possibly reception of mechanical vibrations.

5. The transducer of claim 1 or 4,
**characterized in that** different sectors (5) with receive function, possibly also a receiving piezo-element (11) on the center body (4), are connected for electronic combination of certain undesired frequencies, for noise cancellation.

6. The transducer of claim 1 or 4,
**characterized in that** the surrounding framework (2) is further suspended in an outer frame (6) by means of an outer, elastic suspension structure (7) which is also equipped with piezo-elements to provide receive signals usable for noise cancellation by combination with receive signals from the receiving sectors (5) and possibly a receiving piezo-element (11) on the center body (14).

7. The transducer of claim 1 or 4,
**characterized in that** sectors (5) with receive function are also operative to receive electrical signals from a special external sensor, for cancellation of certain signals that constitute undesired noise.

8. The transducer of claim 1,
**characterized by** equipment attached for phasing signals to and from the sectors (5), for achieving directional transmission and reception.

9. The transducer of claim 4,
**characterized in that** the center body (4) has a separate piezo-element (10) for transmitting ultrasound signals in a frequency range 5-10 MHz, and a separate piezo-element (11) for receiving reflections of these signals, for performing an echo Doppler investigation, possibly simultaneously with auscultation use of at least one of the sectors (5) of said suspension structure.

10. The transducer of claim 1,
**characterized in that** said center body (4) is attached tautably to a tautening structure (12, 13, 15, 16, 17) arranged at a rear side of said suspension structure (3), whereby said center body (4) can be pulled towards the rear side to provide tension and a slanted position for said sectors (5).

11. The transducer of claim 10,
**characterized in that** said tautening structure (12, 13, 15, 16, 17) is attached rigidly in the surrounding framework (2).

12. The transducer of claim 10,
**characterized in that** said tautening structure (12, 13, 15, 16, 17) is attached rigidly in an outer frame (6), in which the surrounding framework (2) is suspended by means of an outer elastic suspension structure (7), whereby the sectors (5) of the suspension structure as well as the outer elastic suspension structure (7) are provided with tension and slanted position when the center body (4) is tautened.

## Patentansprüche

1. Mechano-elektrischer Zweiwegewandler zum Aussenden und Aufnehmen von mechanischen Vibrationen, wobei der Wandler einen Mittelkörper (4), der in mindestens einer piezoelektrischen elastischen Hängestruktur (3) aufgehängt ist, die wiederum in einem umgebenden Rahmentragwerk (2) angebracht ist, und Signalleiter (8), die mit der Hängestruktur verbunden sind, umfasst,
**dadurch gekennzeichnet, dass** die Hängestruktur (3) und der Mittelkörper (4) durch Sektordivision der Hängestruktur (3) für im wesentlichen gleichzeitige Übertragung von mechanischen Vibrationen und Empfang solcher Vibrationen angepasst sind, wobei die Hängestruktur (3) getrennte Signalleiter für jeden Sektor (5) aufweist.

2. Wandler nach Anspruch 1, **dadurch gekennzeichnet, dass** mindestens ein Sektor (5) der Hängestruktur (3) zur Übertragung zur gleichen Zeit funktionsfähig ist, zu der mindestens ein anderer Sektor (5) ein auf dieser Übertragung basierendes Echosignal empfängt.

3. Wandler nach Anspruch 2, **dadurch gekennzeichnet, dass** er zur Verwendung auf eine solche Weise angepasst ist, dass der Mittelkörper (4) während einer Untersuchung auf eine solche Weise, dass Signalübertragung und Signalempfang gerichteter sind, gegen eine Gewebeoberfläche gepresst werden kann, wodurch den Sektoren (5) eine Spannung verliehen wird, während die Sektoren gleichzeitig geneigt werden.

4. Wandler nach Anspruch 1, **dadurch gekennzeichnet, dass** der Mittelkörper (4) mit mindestens einem Piezoelement (10, 11) zum Übertragen und möglicherweise Aufnehmen von mechanischen Vibrationen ausgerüstet ist.

5. Wandler nach Anspruch 1 oder 4, **dadurch gekennzeichnet, dass** verschiedene Sektoren (5) mit Empfangsfunktion, möglicherweise auch ein empfangendes Piezoelement (11) am Mittelkörper (4), zur elektronischen Kombination von bestimmten unerwünschten Frequenzen zur Rauschunterdrückung verbunden sind.

6. Wandler nach Anspruch 1 oder 4, **dadurch gekennzeichnet, dass** das umgebende Rahmentragwerk (2) weiterhin durch eine äußere, elastische Hängestruktur (7), die auch, um Empfangssignale bereitzustellen, die durch Kombination mit Empfangssignalen von dem empfangenden Sektoren (5) zur Rauschunterdrückung verwendbar sind, mit Piezoelementen und möglicherweise mit einem empfangenden Piezoelement (11) am Mittelkörper (14) ausgerüstet ist, in einem äußeren Rahmen (6) aufgehängt ist.

7. Wandler nach Anspruch 1 oder 4, **dadurch gekennzeichnet, dass** Sektoren (5) mit Empfangsfunktion auch funktionsfähig sind, um elektrische Signale von einem speziellen externen Sensor zur Unterdrückung von bestimmten Signalen, die unerwünschtes Rauschen darstellen, zu empfangen.

8. Wandler nach Anspruch 1, **gekennzeichnet durch** Ausrüstung, die zum Erreichen von gerichteter Übertragung und von Empfang zum Einphasen von Signalen an die und von den Sektoren (5) angebracht ist.

9. Wandler nach Anspruch 4, **dadurch gekennzeichnet, dass** der Mittelkörper (4) ein getrenntes Piezoelement (10) zum Übertragen von Ultraschallsignalen in einem Frequenzbereich von 5-10 MHz und ein getrenntes Piezoelement (11) zum Empfangen von Reflexionen dieser Signale zum Durchführen einer Echo-Doppler-Untersuchung, möglicherweise gleichzeitig mit Auskultationsverwendung von mindestens einem der Sektoren (5) der Hängestruktur aufweist.

10. Wandler nach Anspruch 1, **dadurch gekennzeichnet, dass** der Mittelkörper (4) spannbar an einer Spannstruktur (12, 13, 15, 16, 17) angebracht ist, die an einer Rückseite der Hängestruktur (3) angeordnet ist, wodurch der Mittelkörper (4) zur Rückseite gezogen werden kann, um für Spannung und eine geneigte Position für die Sektoren (5) zu sorgen.

11. Wandler nach Anspruch 10, **dadurch gekennzeichnet, dass** die Spannstruktur (12, 13, 15, 16, 17) starr im umgebenden Rahmentragwerk (2) befestigt ist.

12. Wandler nach Anspruch 10, **dadurch gekennzeichnet, dass** die Spannstruktur (12, 13, 15, 16, 17) fest in einem äußeren Rahmen (6) befestigt ist, in dem das umgebende Rahmentragwerk (2) durch eine äußere elastische Hängestruktur (7) aufgehängt ist, wodurch für die Sektoren (5) der Hängestruktur wie auch die äußere elastische Hängestruktur (7) für Spannung und geneigte Position gesorgt wird, wenn der Mittelkörper (4) gespannt wird.

## Revendications

1. Transducteur mécanoélectrique bidirectionnel pour émission et lecture de vibrations mécaniques, le transducteur comprenant un corps central (4) suspendu dans au moins une structure de suspension élastique piézoélectrique (3) qui est elle-même montée dans un cadre périphérique (2) et des conducteurs de signaux (8) reliés à la structure de suspension,
**caractérisé en ce que** la structure de suspension (3) et le corps central (4) sont adaptés à une émission de vibrations mécaniques et à la réception de ces vibrations de façon sensiblement simultanée, par division sectorielle de la structure de suspension (3), la structure de suspension (3) comportant des conducteurs de signal séparés pour chaque secteur (5).

2. Transducteur selon la revendication 1, **caractérisé en ce qu'**au moins un secteur (5) de la structure de suspension (3) agit pour émission en même temps qu'au moins un autre secteur (5) reçoit un signal d'écho basé sur cette émission.

3. Transducteur selon la revendication 2, **caractérisé en ce qu'**il est adapté à être utilisé de sorte que le corps central (4) peut être forcé contre la surface d'un tissu pendant un examen, d'où il résulte qu'une tension est impartie auxdits secteurs (5) tandis que, en même temps, les secteurs deviennent obliques de telle sorte que la transmission de signal et la réception de signal sont plus directionnelles.

4. Transducteur selon la revendication 1, **caractérisé en ce que** le corps central (4) est équipé d'au moins un élément piézoélectrique (10, 11) pour émission et éventuellement réception de vibrations mécaniques.

5. Transducteur selon la revendication 1 ou 4, **caractérisé en ce que** les différents secteurs (5) ayant une fonction de réception, et également éventuellement un élément piézoélectrique récepteur (11) sur le corps central (4), sont connectés pour une combinaison électronique de certaines fréquences indésirées, pour suppression du bruit.

6. Transducteur selon la revendication 1 ou 4, **caractérisé en ce que** le cadre périphérique (2) est en outre suspendu à l'intérieur d'un cadre externe (6) au moyen d'une structure de suspension élastique externe (7) qui est également équipée d'éléments piézoélectriques pour fournir des signaux de réception utilisable pour la suppression du bruit par combinaison avec des signaux de réception en provenance des secteurs récepteurs (5) et éventuellement un élément piézoélectrique récepteur (11) sur le corps central (14).

7. Transducteur selon la revendication 1 ou 4, **caractérisé en ce que** des secteurs (5) à fonction de réception agissent également pour recevoir des signaux électriques à partir d'un capteur externe particulier, pour suppression de certains signaux qui constituent un bruit indésiré.

8. Transducteur selon la revendication 1, **caractérisé par** un équipement lié pour mettre en phase des signaux en direction et à partir des secteurs (5) pour réaliser une émission et une réception directionnelles.

9. Transducteur selon la revendication 4, **caractérisé en ce que** le corps central (4) comporte un élément piézoélectrique séparé (10) pour émission de signaux ultrasonore dans une plage de fréquence de 5 à 10 MHz et un élément piézoélectrique séparé (11) pour recevoir des réflexions de ces signaux, pour réaliser un examen Doppler d'écho, éventuellement en même temps que l'utilisation en auscultation d'au moins un des secteurs (5) de la structure de suspension.

10. Transducteur selon la revendication 1, **caractérisé en ce que** le corps central (4) est fixé rigidement à une structure de raidissement (12, 13, 15, 16, 17) disposée à l'arrière de la structure de suspension (3), d'où il résulte que le corps central (4) peut être tiré vers l'arrière pour assurer une tension et une position inclinée des secteurs (5).

11. Transducteur selon la revendication 10, dans lequel la structure de raidissement (12, 13, 15, 16, 17) est fixée rigidement au cadre environnant (2).

12. Transducteur selon la revendication 10, **caractérisé en ce que** la structure de raidissement (12, 13, 15, 16, 17) est fixée rigidement dans un cadre externe (6), le cadre périphérique (2) étant suspendu au moyen d'une structure de suspension élastique externe (7), d'où il résulte que les secteurs (5) de la structure de suspension ainsi que la structure de suspension élastique externe (7) sont munis d'une tension et prennent une position inclinée quand le corps central (4) est raidi.
